# EUROPEAN PATENT APPLICATION

(11) **EP 4 810 141 A1**
(43) Date of publication of application: **23.09.2026**
(21) Application number: 25861258.9
(22) Date of filing: 25.08.2025
(51) Int. Cl.: B01D 9/00, B01D 17/00, B01D 17/04, B01D 17/02, C07C 51/43, C07C 51/47, C07C 57/04, C07B 63/00

(54) **INTEGRATED PURIFICATION DEVICE FOR CRYSTALLIZATION AND SOLID-LIQUID SEPARATION**

(30) Priority: 28.08.2024 KR 20240115767; 28.08.2024 KR 20240115785; 28.08.2024 KR 20240115803; 20.08.2025 KR 20250115557
(71) Applicant: LG Chem, Ltd., Seoul 07336 (KR); Korea University Research and Business Foundation, Seoul 02841 (KR)
(72) Inventor: YOO, Sung Jin, Seoul 07796 (KR); KIM, Tae Hyun, Seoul 02577 (KR); KANG, Jeong Won, Seoul 02800 (KR); LEE, Sung Kyu, Seoul 07796 (KR); JANG, Kyung Soo, Seoul 07796 (KR)
(74) Representative: Goddar, Heinz J.
(86) International application number: PCT/KR2025/012889
(87) International publication number: WO 2026/049440

(57) **Abstract**

Provided are an integrated purification device including: a crystallizer including an upper area of the crystallizer which is supplied with a compound solution to form crystals and a lower area of the crystallizer which is provided under the upper area of the crystallizer and stores and discharges a slurry including the crystals formed in the upper area of the crystallizer; and a solid-liquid separator including a cylindrical body including a slurry inlet which is provided in one side and supplied with the slurry from the crystallizer and an outlet which is provided in the other side and discharges ground crystals obtained by performing separation from the slurry and then performing compression and washing, wherein the cylindrical body is divided into a first area, a second area, and a third area in order from one side of the cylindrical body, and a purification method using the purification device.

## Description

### [Technical Field]

### CROSS-REFERENCE TO RELATED APPLICATIONS

The present application claims the benefit of priorities to Korean Patent Application No. 10-2024-0115767, filed on August 28, 2024, Korean Patent Application No. 10-2024-0115785, filed on August 28, 2024, Korean Patent Application No. 10-2024-0115803, filed on August 28, 2024, and Korean Patent Application No. 10-2025-0115557, filed on August 20, 2025, the entire contents of which are incorporated herein as a part of the specification.

### Technical Field

The present invention relates to an integrated purification device combining a crystallizer and a solid-liquid separator.

### [Background Art]

Compounds used as raw materials of various resins may be prepared by obtaining a compound-containing solution through a synthesis reaction and then performing a subsequent process of purifying the compound from the solution.

For example, an acrylic acid undergoes a gas phase oxidation reaction with propane, propylene, and the like in the presence of an appropriate catalyst in a reactor to obtain an acrylic acid-containing gaseous stream, which is then subjected to an absorption process to bring it into contact with water in an absorption tower to obtain an acrylic acid aqueous solution. Thereafter, the acrylic acid aqueous solution may undergo crystallization and solid-liquid separation for purifying an acrylic acid.

The crystallization is a process of precipitating a compound as a solid using a difference in solubility of a material depending on temperature in a liquid mixture, and a solution in which the compound is dissolved in a solvent is converted into a suspension in a slurry form in which a solid crystal compound floats in a residual liquid (mother liquor).

The solid-liquid separation is a process for separating compound crystals formed in the crystallization process with a residual liquid and may be performed using devices such as a washing column, a centrifuge, and filter. In particular, since the washing column is effective for separation of the compound crystals and the mother liquor and also may remove the residual liquid and impurities included in the separated compound crystals by washing, it may be used in a process requiring high purity and ultrahigh purity.

### [Disclosure]

### [Technical Problem]

The present invention is intended to solve the problems mentioned in the Background Art, and an object of the present invention is to provide a purification device which may solve a problem of pipe blockage which occurs in slurry transfer in compound crystallization and solid-liquid separation process, and a method for preparing a compound in a high yield.

### [Technical Solution]

In one general aspect, an integrated purification device includes: a crystallizer including an upper area of the crystallizer which is supplied with a compound solution to form crystals and a lower area of the crystallizer which is provided under the upper area of the crystallizer and stores and discharges a slurry including the crystals formed in the upper area of the crystallizer; and a solid-liquid separator including a cylindrical body including a slurry inlet which is provided in one side and supplied with the slurry from the crystallizer and an outlet which is provided in the other side and discharges washed crystals obtained by performing separation from the slurry and then performing compression and washing, wherein the cylindrical body is divided into a first area, a second area, and a third area in order from one side of the cylindrical body, the first area includes an internal screw which moves the slurry supplied to the slurry inlet in a direction of the second area, the second area includes a filter which separates the crystals from the slurry and a scraping knife which grinds the washed crystals to form ground crystals, and the third area includes an outlet which discharges the ground crystals out of the cylindrical body.

In another general aspect, a purification method, which is a method for purifying and recovering crystals from a solution completing a reaction in a reactor using the purification device, includes: (S1) supplying a compound solution after a reaction in a reactor to an upper area of a crystallizer and crystallizing the compound to form a slurry including crystals, and supplying the slurry through a lower area of the crystallizer to a first area of a solid-liquid separator, (S2) moving the slurry supplied to the first area of the solid-liquid separator to a second area by an internal screw, separating the crystals and a mother liquor, respectively, from the slurry through a filter provided in the second area, and discharging the mother liquor out of the solid-liquid separator, (S3) compressing the crystals separated by the filter in the second area of the solid-liquid separator to form a crystal bed, washing the crystal bed with a concentrate to form washed crystals, and grinding the washed crystals with a scraping knife to form ground crystals, (S4) discharging the ground crystals in a third area of the solid-liquid separator out of the solid-liquid separator through an outlet, and supplying the ground crystals to a heat supply device to melt the crystals to form the concentrate, and (S5) supplying at least a part of the concentrate to the third area, wherein a pressure of the third area is formed to be more than 1 bar and no more than 2 bar, and the concentrate is supplied in the direction of the second area by overpressure formed by the pressure of the third area to wash the crystal bed.

### [Advantageous Effects]

In the purification device according to the present invention, a slurry including crystals formed in the crystallizer 20 is supplied to the solid-liquid separator 30, the crystals and a mother liquor are separated in the solid-liquid separator 30, and the crystals are washed, so that purified crystals from which impurities such as the mother liquor have been removed may be obtained.

The solid-liquid separator 30 is divided into an area supplied with the slurry including crystals supplied from the crystallizer, an area separating the crystals from the mother liquor and washing the crystals, and an area discharging the washed crystals, in order, respectively, so that the slurry may pass through the areas sequentially to obtain a final product by a simple method.

In addition, in the solid-liquid separator 30, an internal screw, a filter, and a scraping knife are provided inside, so that a process of separating the slurry into the crystals and the mother liquor, respectively, and collecting and discharging the crystals may be performed.

In addition, in the solid-liquid separator 30, the crystals are washed to remove impurities such as the mother liquor, thereby obtaining a higher-purity final product.

In addition, in the purification device according to the present invention, a lower area of the crystallizer is formed in a cone shape and is combined with the solid-liquid separator, thereby performing crystallization and solid-liquid separation of the compound in one device when purifying the compound solution obtained by the synthesis reaction in the reactor.

Therefore, when a device integrated by combining the crystallizer and the solid-liquid separator is formed, there is no need to have a separate pipe and pump for transferring the slurry between them, and thus, problems such as compound loss and pipe blockage, which are caused in a slurry transfer process in conventional technology, may be solved.

In addition, the slurry is uniformly distributed using oxygen to derive smooth discharge so that the slurry does not accumulate in a combining site of the crystallizer and the solid-liquid separator, thereby preventing a blockage phenomenon in the combining part.

Moreover, a third area of the solid-liquid separator is maintained at more than 1 bar and no more than 2 bar, thereby improving crystal purity by a washing effect without a need for loss of a washing liquid and an increase in device size.

Furthermore, an installation area and installation cost may be decreased by one integrated device, and also, process simplification may be implemented.

### [Description of Drawings]

FIG. 1 illustrates a purification device according to an exemplary embodiment of the present invention and a process of purifying crystals from a compound solution using the device.
FIG. 2 illustrates a solid-liquid separator included in the purification device according to an exemplary embodiment of the present invention.
FIG. 3 is a cross-section of a width of the solid-liquid separator according to an exemplary embodiment of the present invention and illustrates a filter and a mother liquor discharge pipe.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

In the present application, the term "stream" may refer to a fluid flow in the process or may refer to the fluid itself flowing in a moving line (pipe). Specifically, the "stream" may refer to both a fluid itself flowing in a pipe connecting each device and a fluid flow. In addition, the fluid may refer to inclusion of any one or more of gas, liquid, and solid.

In addition, the terms such as "comprise", "contain", "being provided with", or "have" are intended to specify the presence of stated features, numerals, steps, operations, constituent elements, parts, or a combination thereof in the present application, but do not preclude the presence or addition possibility of one or more other features, numerals, steps, operations, constituent elements, parts, or a combination thereof.

Also, when it is described that a constituent element is "connected to", "supported on", or "in contact with" other constituent elements, it includes not only the case in which the constituent elements are directly connected, supported, or in contact, but also the case in which they are indirectly connected, supported, or in contact through a third constituent element.

An exemplary embodiment of the present invention relates to a purification device which may perform crystallization and solid-liquid separation of a compound in one device.

Hereinafter, the specific exemplary embodiment of the present invention will be described in detail with reference to the drawings.

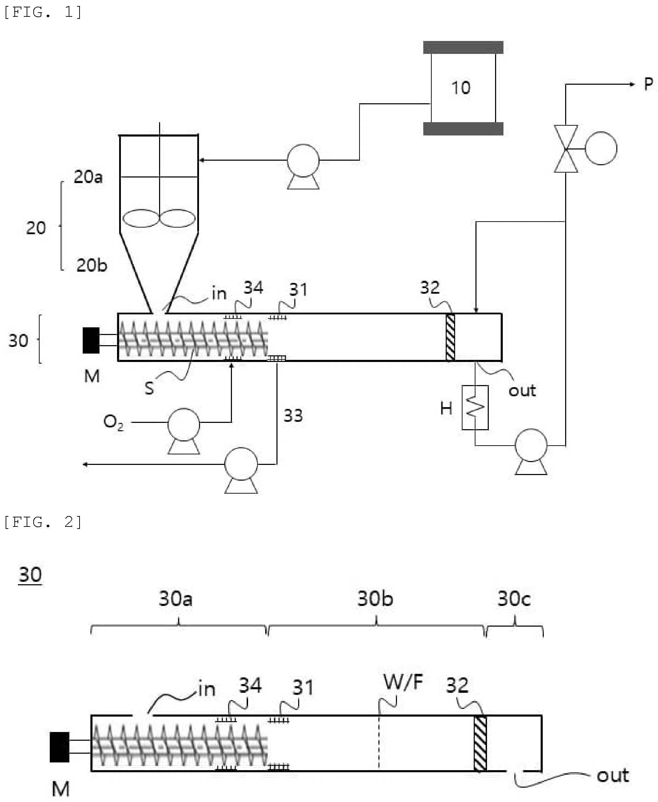

FIG. 1 illustrates the purification device according to an exemplary embodiment of the present invention and a purification process of performing crystallization and solid-liquid separation of a compound solution using the device.

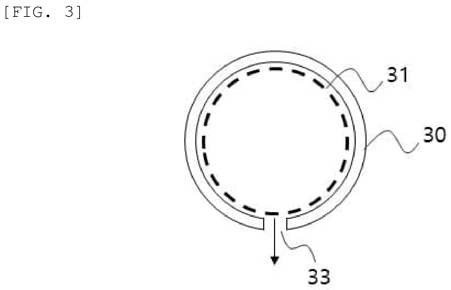

FIG. 2 shows a solid-liquid separator included in the purification device according to an exemplary embodiment of the present invention in detail, and FIG. 3 shows a form in which a filter and a mother liquor outlet are provided in the solid-liquid separator in detail. In FIG. 3, the arrow indicates a direction in which a mother liquor passes through a filter and is discharged through a mother liquor discharge pipe 33.

According to the drawing, the purification device of the present invention includes: a crystallizer including an upper area of the crystallizer which is supplied with a compound solution to form crystals and a lower area of the crystallizer which is provided under the upper area of the crystallizer and stores and discharges a slurry including the crystals formed in the upper area of the crystallizer; and a solid-liquid separator including a cylindrical body including a slurry inlet which is provided in one side and supplied with the slurry from the crystallizer and an outlet which is provided in the other side and discharges washed crystals obtained by performing separation from the slurry and then performing washing, wherein the cylindrical body is divided into a first area, a second area, and a third area in order from one side of the cylindrical body, the first area includes an internal screw which moves the slurry supplied to the slurry inlet in a direction of the second area, the second area includes a filter which separates the crystals from the slurry and a scraping knife which grinds the washed crystals to form ground crystals, and the third area includes an outlet which discharges the ground crystals out of the cylindrical body.

Referring to FIG. 1, the purification device of the present invention may include the crystallizer 20 and the solid-liquid separator 30, and, if necessary, further include a feed tank 10 which supplies a compound solution to the crystallizer 20. The crystallizer 20 may be divided into an upper area 20a of the crystallizer which is provided with a stirrer to form crystals from the compound solution supplied from the feed tank 10 and a lower area 20b of the crystallizer which supplies a slurry including the crystals formed in the crystallizer 20 to the solid-liquid separator 30.

In the crystallizer 20, specifically, the lower area 20b of the crystallizer is provided under the upper area 20a of the crystallizer, so that the slurry formed in the upper area 20a of the crystallizer may move to the lower area 20b of the crystallizer. Herein, the upper area 20a of the crystallizer which is supplied with the feed and performs crystallization includes the stirrer and may be formed in a barrel shape having the same width cross-section so that the stirrer may stir. In addition, the lower area 20b of the crystallizer may be formed in a barrel shape like the upper area of the crystallizer or may be formed in a cone shape having a narrowing width downwardly in order to collect the slurry well. Herein, the lower area 20b of the crystallizer may be a lower part of an area where the stirrer rotates, or when the lower area 20b of the crystallizer has a cone shape, may refer to a lower part of the area where the stirrer rotates and from a part where the width of a barrel shape begins to narrow to a part below.

In an exemplary embodiment, the upper area 20a of the crystallizer may crystallize a specific component into a solid using a solubility difference depending on a temperature for the components included in the compound solution. Specifically, in the upper area 20a of the crystallizer, the compound solution transferred from the feed tank 10 is supplied, and crystallization is performed while flowing the compound solution by the stirrer, thereby obtaining a slurry including the crystals of the compound included in the compound solution.

The compound solution supplied to the upper area 20a of the crystallizer refers to a solution containing a compound produced by the synthesis reaction in a random reactor. As an example, the compound solution may be a feed supplied to the crystallizer, and specifically an acrylic acid aqueous solution obtained by an absorption process of performing a gas phase oxidation reaction of propane, propylene, and the like in the presence of an appropriate catalyst in a reactor to obtain an acrylic acid-containing gaseous stream, which was then brought into contact with water in an absorption tower. Also, a solution containing a compound used as a raw material of various resins may be applied as the compound solution of the present invention.

In addition, when the compound solution is the acrylic acid aqueous solution, the crystals may be acryl crystals formed by crystallizing the acrylic acid aqueous solution.

In addition, the slurry obtained in the upper area 20a of the crystallizer refers to a suspension in which the compound included in the compound solution is precipitated as solid crystals and floats in the liquid mother liquor.

The stirrer installed inside the upper area 20a of the crystallizer may have a shape including a plurality of wings, and crystal formation and crystal growth may be derived while stirring the compound solution during crystallization.

In an exemplary embodiment of the present invention, the stirrer provided in the upper area 20a of the crystallizer may be formed in a shape including a plurality of wings, and a stirring speed of the stirrer affects crystal growth to determine the size of the formed crystals. Considering the fact, the stirrer may be operated at a speed of 5 to 100 rpm or 10 to 80 rpm. When the speed of the stirrer is less than 5 rpm, the circulation of the produced crystal slurry may be limited, and when the speed is more than 100 rpm, the compound crystals included in the slurry may be broken.

In addition, the wings of the stirrer may have a slope, and the slurry obtained in the upper area 20a of the crystallizer during the rotation of the stirrer may move to the lower area 20b of the crystallizer by gravity. More specifically, the wings of the stirrer may be provided and rotate with a slope at an angle of 30° to 50°, for example, 45°, and during the rotation, a fluid is forced in the direction of gravity and the crystals produced in the fluid descend due to gravity with increased density. The inclination angle of the wings of the stirrer may be an angle based on the direction of gravity with respect to the ground, and also, an angle based on a horizontal cross-section of the upper area 20a of the crystallizer.

Meanwhile, the upper area 20a of the crystallizer may be formed to maintain a constant liquid level. For example, from the upper area 20a of the crystallizer to the third area 30c of the solid-liquid separator 30 may be filled with a liquid without an empty space. Specifically, the compound solution supplied from the feed tank 10 in the front stage to the upper area 20a of the crystallizer using a pump is continuously supplied in a combined amount of an amount of the mother liquor discharged through a filter in the solid-liquid separator 30 in the subsequent stage and an amount of the final product (P) discharged through a product discharge pipe, thereby maintaining the liquid level of the crystallizer 20 constant. That is to say, the amount of the compound solution added to the crystallizer 20 and the amount of the mother liquor discharged from the solid-liquid separator 30 and the final product (P) (wherein the amount of the final product refers to an amount obtained by subtracting an amount of a concentrate supplied to the third area 30c from an amount of the washed crystals discharged from the outlet (Out)) are maintained constant to maintain the liquid level of the crystallizer 20, which allows continuous operation of the crystallizer and the solid-liquid separator in a stead state.

In addition, a crystallization temperature may vary depending on the concentration of the compound solution supplied to the upper area 20a of the crystallizer. For example, the higher the concentration of the compound in the supplied compound solution, the higher the crystallization temperature, and the lower the concentration of the compound included in the compound solution, the lower the crystallization temperature. The crystallization temperature may be adjusted to maintain a constant level by installing an outer jacket in the upper area 20a of the crystallizer and circulating a medium such as warm water or cooling water.

It is preferred that the crystallization in the upper area 20a of the crystallizer is performed so that the obtained slurry satisfies a crystal content in a predetermined range. Specifically, the slurry obtained from the crystallization may include 5 to 40 wt% or 10 to 30 wt% of solidified crystals based on the weight of the slurry. When the content of the crystals in the slurry is less than 5 wt%, the yield of the finally obtained crystals is decreased, and when the crystal content is more than 40 wt%, the crystal content in the slurry is increased so that mixing by the stirrer in the upper area 20a of the crystallizer and slurry transfer to the solid-liquid separator 30 through the lower area 20b of the crystallizer may be difficult.

In addition, the compound crystals included in the slurry may have a size of 200 to 800 µm or 300 to 700 µm, and when the size is satisfied, it is favorable for the transfer to the solid-liquid separator 30.

The lower area 20b of the crystallizer is an area where the slurry including the crystals obtained in the upper area 20a of the crystallizer is collected and discharged and may be formed in a barrel shape having a constant width or in a cone shape having a decreasing cross-sectional diameter downwardly. In addition, the lower area 20b of the crystallizer may have a form having an open lower end, and the open-shaped lower end may be inserted into the slurry inlet of the solid-liquid separator and integrated. A joint O-ring for strengthening combining and preventing leakage may be provided between the lower end of the lower area 20b of the crystallizer and the slurry inlet of the solid-liquid separator, and for example, the combining may be made by mounting an O-ring made of a chemical resistant silicone rubber material for leakage prevention and fixing the insertion state.

Thus, since the crystallizer 20 and the solid-liquid separator 30 may be integrated without a separate pipe connecting them, problems such as compound loss or pipe blockage caused in the slurry transfer process in the conventional technology may be solved. In addition, when the crystallizer and the solid-liquid separator are combined and integrated, there is no need to provide a slurry pump required for slurry transfer as well as a pipe connecting them, and an installation area and installation cost may be reduced, and process simplification may be implemented therefrom.

In addition, when the lower area 20b of the crystallizer has a cone shape, the diameter of the lowermost cross-section may be decreased to 0.05 to 0.4 times or 0.1 to 0.3 times the diameter of the uppermost cross-section of the upper area 20a of the crystallizer. When the diameter of the lowermost cross-section of the lower area 20b of the crystallizer is less than 0.05 times the diameter of the uppermost cross-section of the upper area 20a of the crystallizer, a blockage phenomenon may occur when the slurry moves from the lower area 20b of the crystallizer to the solid-liquid separator 30, and when it is more than 0.4 times, an area where the slurry is added to the solid-liquid separator 30, that is, a slurry inlet area is increased to lower compression efficiency of crystals in the slurry by the internal screw.

In the integrated purification device of the present invention, the solid-liquid separator 30 may be combined in a horizontal or vertical direction with the lower area 20b of the crystallizer. As an example, FIG. 1 illustrates an embodiment in which the solid-liquid separator 30 is disposed in a horizontal direction and combined, and in this case, the long side of the solid-liquid separator 30 may be perpendicular to the crystallizer 20.

Referring to the drawing again, in the purification device according to the present invention, the solid-liquid separator 30 includes a cylindrical body, a slurry inlet which is provided in one side of the cylindrical body and supplied with the slurry from the crystallizer, and an outlet which is provided in the other side of the cylindrical body and discharges ground crystals obtained by performing separation from the slurry and then performing compression and washing. The cylindrical body is divided into a first area, a second area, and a third area from one side of the cylindrical body; the first area may include an internal screw which moves the slurry supplied to the slurry inlet in the direction of the second area, the second area may include a filter which separates the crystals from the slurry and a scraping knife which grinds the washed crystals to form ground crystals, and the third area may include an outlet which discharges the ground crystals out of the cylindrical body.

In addition, according to an exemplary embodiment, in the purification device of the present invention, the solid-liquid separator 30 may be combined in a horizontal or vertical direction with the lower area 20b of the crystallizer. As an example, FIG. 1 illustrates an embodiment in which the solid-liquid separator 30 is combined horizontally with the lower area 20a of the crystallizer. In this case, the crystallizer 20 and the long side of the solid-liquid separator 30 may be perpendicular. When the crystallizer 20 and the solid-liquid separator 30 are combined vertically, the slurry (or crystals) supplied to the solid-liquid separator 30 may move from the first area to the third area by the internal screw provided in the inside of the solid-liquid separator 30.

Specifically, the first area 30a of the solid-liquid separator accommodates the slurry introduced through the slurry inlet (In), and the slurry may move in the direction of the second area 30b by the rotation of the internal screw. In addition, the first area 30a may optionally include an oxygen input port 34 in order to make the introduction and movement of the slurry well, and oxygen may be introduced through the oxygen input port connected to an external oxygen supply line.

The internal screw (S) has a shape having spiral wings provided based on a central axis and may rotate by receiving power from a motor (M) to move the slurry in the direction of the second area 30b. The internal screw (S) may rotate by axial rotation of the spiral wings based on the central axis.

The internal screw may have a length from one end of the solid-liquid separator 30 to the boundary between the first area and the second area, or from one end of the solid-liquid separator 30 to the area where the filter is provided. This may be a length which allows the internal screw to effectively move the slurry to the area with the filter and may transfer sufficient power to the slurry and the crystals (or crystal bed and washed crystals).

The internal screw may move the slurry and compress the crystals while rotating at 5 to 100 rpm or 10 to 80 rpm considering the pressure of the concentrate recirculating to the third area 30c. When the rotation speed of the internal screw is less than 5 rpm, a force to push the crystals inside the solid-liquid separator 30 is insufficient, so that the crystal bed may not be sufficiently compressed. Meanwhile, when the rotation speed of the internal screw is more than 100 rpm, an excessive force is applied to the crystals to cause agglomeration during crystal compression, and an impermeable material layer is formed in the crystal bed to make the operation difficult. The impermeable material layer means that it is excessively compressed so that the concentrate does not wash the crystal bed while passing through the crystal bed, and the mother liquor between the crystal beds does not escape. Therefore, when the impermeable material layer is formed, the crystal bed is not sufficiently washed and the purity of the washed crystals may be lowered.

In addition, the first area 30a may further include the oxygen input port 34.

The oxygen input port 34 has an opening from which oxygen may come and is provided to surround the inner wall of the cylindrical body, but is connected to the oxygen supply line outside to supply oxygen to the first area 30a. The oxygen input port 34 is for preventing slurry accumulation in a connection part between the crystallizer 20 and the solid-liquid separator 30 by supplying oxygen to the first area 30a while introducing and moving the slurry in the first area 30a of the solid-liquid separator.

Specifically, the oxygen input port 34 is connected to an external oxygen supply line and may supply a certain amount of oxygen through a pump installed in the oxygen supply line. The oxygen supplied to the oxygen input port 34 forms bubbles in the slurry and moves in the direction of the crystallizer 20 by a pressure difference between the crystallizer 20 under atmosphere pressure, and the second area and the third area which are the right area of the solid-liquid separator 30 where overpressure is formed as described later, and prevents slurry accumulation in the connection part between the crystallizer 20 and the solid-liquid separator 30 to derive uniform distribution and smooth discharge of the slurry.

It is preferred that the oxygen for the action is supplied before the slurry forms the crystal bed in the solid-liquid separator 30. Therefore, it is favorable that the oxygen input port 34 is installed near the slurry inlet (In) in the first area 30a of the solid-liquid separator, for example, in the middle position based on the full length of the first area 30a, specifically in a position of 20% to 60% or 30% to 55% of the first area 30a from one side of the solid-liquid separator. In addition, the oxygen input port 34 should be installed so that slurry movement is not impeded inside the solid-liquid separator 30, and for example, may be formed in the inner wall surface of the cylindrical body, as shown in FIG. 2.

The range of the first area 30a may be a range to accommodate the internal screw having a length at which power to move the slurry, the crystal bed, and the washed crystals may be transferred. The first area 30a of the solid-liquid separator may occupy the area corresponding to 10 to 60% or 20 to 50% of the full length of the solid-liquid separator.

Meanwhile, specifically, the solid-liquid separator 30 may include the second area. The second area 30b of the solid-liquid separator is a middle area of the solid-liquid separator and may be positioned between the first area 30a and the third area 30c. In the second area 30b, the front end close to the first area 30a may be provided with the filter 31, and the rear end close to the third area 30c may be provided with the scraping knife 32.

Specifically, referring to FIG. 3, the filter 31 is provided along the inner wall surface of the cylindrical body while surrounding the inner wall surface of the cylindrical body, and may separate the slurry into the crystals and the mother liquor. In addition, the mother liquor separated through the filter 31 may be discharged out of the solid-liquid separator 30, and the crystals may move in the direction of the third area 30c by the internal screw. The crystals and the mother liquor may be separated from the slurry in this manner, and the mother liquor passing through the filter 31 may be discharged out of the solid-liquid separator 30 through the mother liquor discharge pipe 33 connected to the filter 31. Herein, a pump for adjusting the discharge amount of the mother liquor may be installed in the mother liquor discharge pipe 33.

The filter may be provided in the direction of the second area 30b, starting from the boundary between the first area 30a and the second area 30b.

The crystals are separated from the mother liquor through the filter 31 and compressed to form the crystal bed. In the second area 30b after the area where the filter is provided, most of the mother liquor is discharged through the filter and the amount of the crystals is increased, and the crystals may be compressed by the force pushed by the internal screw to form the crystal bed. The crystal bed fills the internal space of the second area 30b and may move in the direction of the third area 30c by the force pushed by the internal screw. Herein, since the crystal bed is compressed so that the impermeable material layer is not formed as described above, impurities including a small amount of the mother liquor remaining between the crystals forming the crystal bed may be included, and in order to remove the impurities, the crystal bed needs to be washed.

To this end, the crystal bed compressed in the second area 30b is washed by the concentrate supplied from the third area 30c to form the washed crystals. The concentrate is formed by melting the ground crystal which is finally discharged from the solid-liquid separator 30, and may be discharged through the outlet (Out) provided with a product discharge valve to obtain a final product (P), but at least a part of the concentrate may be recirculated to the third area 30c and used to wash the crystal bed. The concentrate is supplied to the third area 30c to form overpressure in the third area 30c and may flow in the opposite direction of crystal bed movement, that is, in the direction from the third area 30c to the second area 30b and wash impurities including the residual mother liquor remaining in the crystal bed. The overpressure refers to a pressure higher than atmospheric pressure and may be formed by a flow rate of a solution which is recirculated to the third area 30c through the amount of the crystal bed moving from the second area 30b to the third area 30c by the internal screw and the valve adjustment of the final product discharge line. That is to say, when the pressure formed by the flow rate of the concentrate supplied to the third area 30c is higher than the pressure formed in the second area 30b, overpressure is formed in the third area 30c, and the concentrate may wash the crystal bed while moving in the direction of the second area 30b by a pressure difference due to the overpressure. The washed crystals formed by washing with the concentrate may have significantly decreased impurities and high purity.

While the concentrate is supplied in the direction of the second area 30b by the overpressure, a reverse flow in the opposite direction to the progression direction of the crystal bed may be formed. Herein, in the second area 30b, a wash front (W/F) which is a boundary between the impurities including the residual mother liquor and the concentrate may be produced. The impurities may include the mother liquor which is not discharged through the filter and may be discharged through the filter by formation of the reverse flow by the pressure of the washing liquid supply.

The position of the wash front may be formed between the filter and the scraping knife in the second area 30b and the position may be adjusted by the overpressure. For example, when the overpressure is strongly formed, the wash front is formed close to the filter, and when the overpressure is weakly formed, the wash front may be formed close to the scraping knife. It is preferred that the position of the wash front is formed close to the center between the filter and the scraping knife. When the wash front is formed too close to the filter, the concentrate may be discharged to the outside through the filter so that loss may occur, and when the wash front is formed too close to the scraping knife, the crystal bed is not sufficiently washed so that the purity of the concentrate (or final product (P)) may be lowered. The residual mother liquor may be removed from the crystals accumulated in the space of the wash front and the scraping knife 32 to increase the crystal purity.

In the crystal bed washed by the concentrate, the washed crystal may be formed. That is, the crystals present in the direction of the third area 30c from the wash front may be washed crystals from which the impurities have been removed. The concentrate may be included between the crystals forming the washed crystals, but is formed by melting after grinding the washed crystals and does not affect the purity. That is, the impurities may be removed without affecting the purity of the washed crystals, by washing the crystal bed with the concentrate.

Thereafter, the washed crystal may be ground by mechanical action by rotation of the scraping knife 32 and then moved to the third area 30c.

The scraping knife 32 may be positioned in the second area 30b from the boundary between the second area 30b and the third area 30c. In the scraping knife 32, a plurality of blades from the center of the cylindrical body may be formed toward the wall surface of the cylindrical body. Specifically, the scraping knife may be a knife in the shape of a fan or rotating disk having blades provided inside the cylindrical body. While the scraping knife 32 rotates in place, the washed crystals are ground by the blades to form the ground crystals.

In the inside of the solid-liquid separator 30, the slurry may move in the direction of the third area (or second area) by the rotation of the internal screw, the crystal bed also may move in the direction of the third area by the force pushed by the slurry, and the washed crystals and the ground crystals may also move to the third area 30c by the force pushed by the crystal bed.

The second area 30b may occupy the area corresponding to the length of 20 to 70% or 30 to 60% based on the full length of the solid-liquid separator. The starting point of the second area 30b may be a point at which the first area 30a ends.

Meanwhile, the solid-liquid separator 30 may include the third area 30c. The third area is provided in the other side of the cylindrical body and may include an outlet (Out) through which the ground crystals are discharged out of the cylindrical body. The outlet may be connected to the heat supply device to melt the ground crystals.

The ground crystals may be transferred to the heat supply device (H) through the outlet (Out) from the third area 30c. The heat supply device (H) may be a heat exchanger, and when the ground crystals are supplied, the concentrated crystals are melted by heat to form the concentrate, which may be recovered as a purified final product (P).

As described above, at least a part of the concentrate may be recirculated to the third 30c, and the recirculated concentrate may move in the direction of the second area 30b and be used as a washing liquid for removing impurities remaining in the crystal bed. Since the concentrate is a melt of the ground crystals, the purities of the concentrate and the ground crystals may be the same.

The flow rate of the concentrate circulated to the third area 30c of the solid-liquid separator may be controlled so that the content of the washed crystals (or ground crystals) is maintained at an appropriate level.

Meanwhile, the ground crystals may move in the form of a slurry including the ground crystals in the concentrate in order to maintain flowability in the third area 30c, and the flow rate of the concentrate may be controlled to maintain the content of the ground crystals included in the concentrate at an appropriate level.

The flow rate of the concentrate may affect the pressure formed in the third area 30c. The third area 30c of the solid-liquid separator may form an overpressure of more than 1 bar and no more than 2 bar or more than 1 bar and no more than 1.5 bar, in order to maintain a uniform reverse flow of the recirculated concentrate to increase the washing effect. When the overpressure is 1 bar or less, it is difficult to generate the reverse flow of the concentrate, and the wash front is formed close to the scraping knife, so that it may be difficult to expect improvement of purity through the washing effect. Meanwhile, when the overpressure is more than 2 bar, the reverse flow is excessively formed so that the wash front is formed close to the filter, and the concentrate moves toward the filter to cause concentrate loss. In addition, when the overpressure is more than 2 bar, the amount of the recirculated concentrate is increased to decrease the amount of the final product (P), and the size of the purification device for obtaining a desired production amount may be increased, which may be unfavorable in the economic terms.

The third area 30c of the solid-liquid separator may occupy an area corresponding to 3 to 20% or 5 to 15% of the full length of the solid-liquid separator. The starting point of the third area 30b may be a point at which the second area 30a ends.

Additionally, the inside of the solid-liquid separator 30 may be maintained to be insulated for improving smooth movement of the slurry and crystal separation efficiency. For example, the outside of the solid-liquid separator 30 is manufactured in the form of a jacket to form a vacuum state, thereby minimizing heat loss to maintain insulation.

In the purification device of the present invention as described above, the crystals formed in the crystallizer 20 are supplied to the solid-liquid separator 30, the crystals and a mother liquor are separated in the solid-liquid separator 30, and the crystals are washed, thereby obtaining purified crystals from which impurities such as the mother liquor have been removed.

The solid-liquid separator 30 is divided into an area supplied with the slurry including crystals supplied from the crystallizer, an area separating the crystals from the mother liquor and washing the crystals, and an area discharging the washed crystals in order, respectively, so that the slurry may pass through the areas sequentially to obtain a final product by a simple method.

In addition, in the solid-liquid separator 30, the internal screw, the filter, and the scraping knife are provided inside, so that a process of separating the slurry into the crystals and the mother liquor, respectively, and collecting and discharging the crystals may be performed.

In addition, in the solid-liquid separator 30, the crystals are washed to remove impurities such as the mother liquor, thereby obtaining a higher-purity final product.

In addition, in the purification device of the present invention as described above, an integrated device in which the lower area 20b of the crystallizer is configured in a cone shape and combined with the solid-liquid separator 30 may be formed, and crystallization and solid-liquid separation may be performed in one device.

When the crystallizer 20 and the solid-liquid separator 30 are combined, there is no need to have separate pipe and pump for transferring the slurry between them, and thus, problems such as compound loss and pipe blockage, which are caused in a slurry transfer process in conventional technology, may be solved.

In addition, an installation area and installation cost may be decreased by one integrated device, and also, process simplification may be implemented.

In addition, according to an exemplary embodiment, the present invention further provides a purification method of a compound solution which is a solution supplied after completing a reaction in a reactor, using the purification device.

The purification method includes: (S1) supplying a compound solution completing a reaction in a reactor to an upper area of a crystallizer and crystallizing the compound to form a slurry including crystals, and supplying the slurry through a lower area of the crystallizer to a first area of a solid-liquid separator provided with the first area to a third area in order, (S2) moving the slurry supplied to the first area of the solid-liquid separator to a second area by rotating an internal screw, separating the crystals and a mother liquor, respectively, from the slurry through a filter provided in the second area, and discharging the mother liquor out of the solid-liquid separator, (S3) compressing the crystals separated by the filter in the second area of the solid-liquid separator to form a crystal bed, washing the crystal bed with a concentrate to form washed crystals, and grinding the washed crystals with a scraping knife to form ground crystals, (S4) discharging the ground crystals in the third area of the solid-liquid separator out of the solid-liquid separator through an outlet, and supplying the ground crystals to a heat supply device to melt the crystals to form the concentrate, and (S5) supplying at least a part of the concentrate to the third area, wherein a pressure of the third area is formed to be more than 1 bar and no more than 2 bar, and the concentrate is supplied in the direction of the second area by overpressure formed by the pressure of the third area to wash the crystal bed.

In addition, in the purification method according to the present invention, oxygen is introduced to the first area 30a of the solid-liquid separator and moves in the direction of the crystallizer, thereby preventing slurry accumulation in the connection part of the solid-liquid separator 30 and the crystallizer 20.

In the purification device according to an exemplary embodiment of the present invention, the crystallizer 20, the solid-liquid separator 30, and the combination thereof are as described above for the purification device according to the present invention in the overlapping range.

Hereinafter, the present invention will be described in more detail by the examples. However, the following examples are provided for illustrating the present invention, and it is apparent to a person skilled in the art that various modifications and alterations may be made without departing from the scope and spirit of the present invention, and the scope of the present invention is not limited thereto.

### Example 1:

As illustrated in FIG. 1, compound purification was performed, using a device in which a crystallizer 20 and a solid-liquid separator 30 were integrated.

Specifically, an acrylic acid solution including acrylic acid (98 wt%), acetic acid (1 wt%), and water (1 wt%) was supplied to an upper area 20a of the crystallizer as a feed and was crystallized to obtain a slurry including compound crystals, and then the slurry was introduced to a first area 30a of a solid-liquid separator through a cone-shaped lower area 20b of the crystallizer.

While the slurry introduced to the first area 30a of the solid-liquid separator was moved by an internal screw (S), a mother liquor was discharged through a filter 31 included in the second area 30b to separate the crystals. At this time, the internal screw was rotated at 40 rpm.

The crystals separated by the filter 31 in the second area 30b of the solid-liquid separator were compressed by the internal screw to form a crystal bed. The crystal bed was washed with a concentrate transferred from a third area 30c to form washed crystals, and the washed crystals were ground with a scraping knife 32 in a rotating disc shape to form ground crystals, which were moved to the third area 33c. While the pressure of the third area 33c was maintained to be 1.1 to 1.2 bar, the ground crystals were passed through a heat supply device (H) via an outlet (Out) and melted to form the concentrate. A part of the concentrate was supplied to the third area 30c and the rest was obtained as a purified final product (P).

### Examples 2 and 3 and Comparative Example 1:

The process was performed in the same manner as in Example 1, except that the pressure in the third area of the solid-liquid separator was changed as shown in FIG. 1.

**[Table 1]**

| | Pressure (bar) in third area of solid-liquid separator | Composition of final composition | | |
|---|---|---|---|---|
| | | Acrylic acid (wt%) | Acetic acid (wt%) | Water (wt%) |
| Example 1 | 1.1-1.2 | 99.48 | 0.33 | 0.19 |
| Example 2 | 12.-1.3 | 99.5 | 0.27 | 0.23 |
| Example 3 | 1.3-1.4 | 99.63 | 0.21 | 0.16 |
| Comparative Example 1 | 1 | 98.53 | 0.76 | 0.71 |

As shown in Table 1, Examples 1 to 3 in which a pressure of more than 1 bar was maintained in the third area of the solid-liquid separator combined with the crystallizer had improved crystal purity by deriving the reverse flow of the washing liquid by the overpressure, while Comparative Example 1 in which the pressure in the third area was 1 bar and the reverse flow of the washing liquid was not generated had the decreased crystal purity of the acrylic acid included in the final product.

Meanwhile, when the pressure in the third area of the solid-liquid separator was more than 2 bar, the crystal purity of the final product was able to be increased, but the loss of the concentrate moving in the direction from the third area 30c to the second area 30b occurred and the amount of the recirculated concentrate was increased, which caused a limitation which requires increasing the size of the purification device for obtaining a desired production amount and is not preferred.

### [Reference signs list]

10: Feed tank
20: Crystallizer
20a: Upper area of crystallizer
20b: Lower area of crystallizer
30: Solid-liquid separator
30a: First area
30b: Second area
30c: Third area
31: Filter
32: Scraping knife
33: Mother liquor discharge pipe
34: Oxygen input port
S: Internal screw
In: Slurry inlet
Out: Outlet
H: Heat supply device

## Claims

1. An integrated purification device comprising:
a crystallizer including an upper area of the crystallizer which is supplied with a compound solution to form crystals and a lower area of the crystallizer which is provided under the upper area of the crystallizer and stores and discharges a slurry including the crystals formed in the upper area of the crystallizer; and
a solid-liquid separator including a cylindrical body including a slurry inlet which is provided in one side and supplied with the slurry from the crystallizer and an outlet which is provided in the other side and discharges ground crystals obtained by performing separation from the slurry and then performing compression and washing,
wherein the cylindrical body is divided into a first area, a second area, and a third area in order from one side of the cylindrical body, the first area includes an internal screw which moves the slurry supplied to the slurry inlet in a direction of the second area, the second area includes a filter which separates the crystals from the slurry and a scraping knife which grinds washed crystals to form ground crystals, and the third area includes an outlet which discharges the ground crystals out of the cylindrical body.

2. The integrated purification device of claim 1, further comprising a heat supply device connected to the outlet,
wherein the ground crystals are melted in the heat supply device and form a concentrate, and
at least a part of the concentrate is supplied to the third area and washes a crystal bed formed in the second area by compressing the crystals to form the washed crystals.

3. The integrated purification device of claim 2,
wherein a pressure of the third area is formed to be more than 1 bar and no more than 2 bar, and
the concentrate is supplied in the direction of the second area by overpressure formed by the pressure of the third area to wash the crystal bed.

4. The integrated purification device of claim 1,
wherein the lower area of the crystallizer has a cone shape having a narrowing width downward, and
the lower area of the crystallizer having the cone shape has a form combined with the slurry inlet of the solid-liquid separator.

5. The integrated purification device of claim 4, wherein a diameter of a lowermost cross-section of the lower area of the crystallizer is 0.05 to 0.4 times a diameter of an uppermost cross-section of the upper area of the crystallizer.

6. The integrated purification device of claim 1, wherein the slurry formed in the upper area of the crystallizer includes 5 to 40 wt% of the crystals based on the weight of the slurry.

7. The integrated purification device of claim 1,
wherein the first area further includes an oxygen input port, and
the oxygen input port supplies oxygen to the first area to prevent accumulation of the slurry.

8. The integrated purification device of claim 1,
wherein the filter is provided in the second area from a boundary between the first area and the second area, and is provided in a form of surrounding an inner wall of the cylindrical body in a circular shape to separate the slurry into the crystals and a mother liquor, respectively.

9. The integrated purification device of claim 1, wherein the internal screw rotates by a motor provided in one side end of the cylindrical body.

10. The integrated purification device of claim 1, wherein the internal screw rotates at a speed of 5 to 100 rpm.

11. A purification method comprising:
(S1) supplying a compound solution completing a reaction in a reactor to an upper area of a crystallizer and crystallizing the compound to form a slurry including crystals, and supplying the slurry through a lower area of the crystallizer to a first area of a solid-liquid separator provided with the first area to a third area in order,
(S2) moving the slurry supplied to the first area of the solid-liquid separator to a second area by rotating an internal screw, separating the crystals and a mother liquor, respectively, from the slurry through a filter provided in the second area, and discharging the mother liquor out of the solid-liquid separator,
(S3) compressing the crystals separated by the filter in the second area of the solid-liquid separator to form a crystal bed, washing the crystal bed with a concentrate to form washed crystals, and grinding the washed crystals with a scraping knife to form ground crystals,
(S4) discharging the ground crystals in the third area of the solid-liquid separator out of the solid-liquid separator through an outlet, and supplying the ground crystals to a heat supply device to melt the crystals to form the concentrate, and
(S5) supplying at least a part of the concentrate to the third area,
wherein a pressure of the third area is formed to be more than 1 bar and no more than 2 bar, and
the concentrate is supplied in a direction of the second area by overpressure formed by the pressure of the third area to wash the crystal bed.
